# EUROPEAN PATENT APPLICATION

(11) **EP 0 773 031 A1**
(43) Date of publication of application: **14.05.1997**
(21) Application number: 96307812.6
(22) Date of filing: 29.10.1996
(51) Int. Cl.: A61L 2/20

(54) **Rapid purifying method using ozonized water of high concentration**

(30) Priority: 31.10.1995 JP 308256/95
(71) Applicant: Core Corporation, Shimizu-shi, Shizuoka (JP)
(72) Inventor: Murakami, Atsushi, Shimizu-shi, Shizuoka (JP); Akahori, Yukio, Fujieda-shi, Shizuoka (JP)
(74) Representative: West, Alan Harry

(57) **Abstract**

According to the present invention there is provided a purifying method wherein very fine organisms such as protozoa and plankton, or bacteria or virus, or those with which or on which they are symbiotic or parasitic, are subjected to the action of ozonized water for killing, or oils, fats, resins, or other contaminants, adhered to objects are subjected to the action of ozonized water for exfoliation from the objects.

## Description

The present invention relates to a simple and rapid purifying method capable of rapidly purifying complicated pollution which have heretofore been considered difficult or impossible to purify. According to the present invention there is established a sure technique for the production and concentration control of an ozonized water of a high concentration (hereinafter referred to as "high concentration ozonized water") having a concentration exceeding 20 ppm the preparation of which has heretofore been impossible and those properties and applications have therefore been unknown. According to the present invention, moreover, there is created a rapid purifying technique making the most of the characteristics of the said high-concentration ozonized water, and is thereby provided, for example, a novel purifying method whereby very fine organisms such as protozoa and plankton are killed to purify the environment, further, combined bacterial pollution, including symbiosis and parasitism of those organisms with bacteria, viruses, or the like, are purified fundamentally to cut off the infection route, or stubborn stains on object surfaces caused by the adhesion thereto of oils, fats, or resins, are cleansed by decomposition.

It has long been a well-known fact that ozone exhibits a bactericidal action. However, its practical application has not spread yet because ozone is strongly toxic and inconvenient to handle. If it is dissolved in water, the resulting aqueous water ought to be convenient to handle. However, ozone is difficult to dissolve in water, so at an ordinary temperature it is difficult to prepare an ozone solution of several ppm or more in concentration; besides, it is extremely difficult to control its concentration. For this reason, quantitative data superior in reliability have heretofore been almost nil, and therefore the utilization of ozonized water has not been realized yet despite of the excellent performance of ozone.

In view of such circumstances we have first made a basic study about the dissolution of ozone, then developed an ozonized water concentration controlling technique, confirmed the fact that bacteria die out by instantaneous contact thereof with ozonized water of 4 ppm, and could establish and practically apply a disinfection method using ozonized water and capable of assuring the effect of disinfection. This method is effective for any type of bacteria widely irrespective of whether the bacteria possess drug tolerance or not. What is more, this method is simple and highly safe, thus meeting ideal conditions. These are epoch-making effects incapable of being attained by the conventional disinfection technique. The principle and method associated have already been described in the specification of a pending application.

Our subsequent studies have revealed that virus is also inactivated completely by contact thereof in the unit of second with a low-concentration ozonized water, in addition to the effectiveness for bacteria. With conventional drugs, such rapid and perfect effect for virus has been unattainable. Further, an immediate sterilizing effect of a low-concentration ozonized water of 4 ppm or so on amoeba trophozoite, or vegetative hypha, larger morphologically than bacteria, based on instantaneous cell destruction, could also be confirmed. However, against the amoeba cyst having a solid outer wall, the low-concentration ozonized water was not effective. It follows that, except special cases including the presence of such cyst, the low-concentration ozonized water has exhibited an excellent insecticidal, bactericidal and inactivating effect in the unit of second for all of very fine organisms, including protozoa such as amoeba, as well as bacteria and virus.

However, against the amoeba cyst covered with a solid shell and exhibiting a strong resistance, it is necessary to use a high-concentration ozonized water capable of displaying a stronger action. Having made a further study covering such a wider range, we found out that there were a lot of fields requiring the use of a high-concentration ozonized water. Consequently, it became absolutely necessary to make a technical study about the manufacture of a high-concentration ozonized water and a method for its utilization.

The grounds for such anti-amoeba measure having been set as a target are based on the following circumstances.

Amoeba is a kind of protozoa, living in a watery place such as pool, cooling tower for air conditioning, watery portions in a house, etc., and thus it is very likely for us to contact amoeba in our daily life. Even amoeba not having pathogenicity can be a great menace to the human health due to parasitism of a pathogenic bacterium thereon. For example, Legionnaires disease is a disease caused by infection of a gram-negative, polar monotrichous, Legionella strain, and there has been an example of Legionnaires disease having caused a mass outbreak of grave pneumonia up to the degree of a social problem. As to bacteria of this genus, their habitat area extends to artificial environments such as cooling tower for air conditioning and hot water supply system in buildings and hospitals in addition to watery places and soil in the natural world. They are symbiotic with algae and parasitic on protozoa such as amoeba. The conventional disinfectants are effective against bacteria themselves, but are ineffective against algae and protozoa. By the conventional disinfection method, therefore, it is extremely difficult to exterminate those bacteria in symbiotic or parasitic form from their habitat areas such as cooling tower for air conditioning. Thus, new urban type infection sources are generally existent around our daily life, and this is dangerous particularly to old people declined in resistance. On the other hand, reference is made also to the case where amoeba itself possesses pathogenicity like Entamoeba histolytica. In many cases, with a cyst carrier as an infection source, the rage of Entamoeba histolytica is induced by oral infection of a maturation cyst. As to anti-infection drugs for such a disease, the development of anti-amoeba drugs has generally been delayed markedly in comparison with the development of antibacterial drugs. Particularly, in the case of cyst, it has so far been considered very difficult to develop an effective drug because of a strong resistance of cyst.

Under such circumstances, importance is attached from the standpoint of public hygiene to the development of a new technique for causing not only trophozoite but also cyst to die up to thereby exterminate amoeba and purify the entire water area rapidly and thoroughly. But a sure method for achieving this purpose has so far not been developed yet.

It has noted previously that ozonized water destroys cells rapidly in the unit of second. In this first stage the cell membrane destructing action proceeds rapidly, and a sharp and quick destruction against such membrane structure is an unparalleled merit peculiar to ozonized water.

Accordingly, the effectiveness of ozonized water can be expected also for the purpose of removing stains of filmily adhered or applied oils, fats, or resins. The object of application is widely distributed also in various steps in the present-day precision industry, for example in the step of destroying and removing resin films in the semiconductor manufacturing process or in the step of thoroughly purifying the surfaces of materials and parts in the precision industry.

At present, the number of working steps requiring such thorough purification are going on increasing. To cope with this tendency, various methods are used according to purposes such as, for example, the use of a strong acid or a strong alkali, or a combination thereof with an oxidizing agent, e.g. chromic acid or hydrogen peroxide, or washing with a flon type solvent.

The use of these materials involves various problems such as the disposal of effluent after use, fear of environmental pollution, and wasting of a large amount of water after use. Further, these problems also lead to an increase of the product cost.

Under the circumstances, a keen desire has been existent for the development of a rapid purifying technique which permits easy execution of post-treatments, can realize perfect purification and is applicable to many technical fields. It can be expected that the advanced purifying action of a high-concentration ozonized water will exhibit an outstanding effect also in such fields.

Turning now our eyes to environmental problems, the technique for controlling and managing the degree of growth of microorganisms such as animal and vegetable planktons which are concerned with water pollution is an extremely important technique when viewed from the standpoint of environment conservation. In this connection, however, an extremely effective environment purifying technique and an environment control method which utilizes such purifying technique have so far not been established yet. This is because conventional drugs are not sufficiently efficacious for this purpose and besides there arises a new pollution problem attributable to residual drugs. Here again is found a great possibility of utilization of a high-concentration ozonized water.

However, a look at the development of science and technology, including that of recent years, shows that in the ozone application sphere there had been reported, before our study, few quantitative experiments of high reproducibility not only with respect to the foregoing problems involving the applicability of a high-concentration ozonized water but also with respect to the knowledge on the action of a low-concentration ozonized water.

Besides, all of the above subjects encounter difficulties in practical application because they involve various problems such as complicated post-treatment and increase of costs which are encountered in the conventional methods. Therefore, the development of a new method capable of overcoming those drawbacks, namely a new method which permits easy execution of a post-treatment and which can achieve a satisfactory effect in a rapid, simple and assured manner, is an extremely important demand in the present day.

The present invention has been accomplished in view of the present situation mentioned above and it is a subject of the invention to establish a thorough and rapid purifying technique using a high-concentration ozonized water and having much more excellent features than before and thereby meet the many years earnest desire in this industry.

According to the present invention, in order to attain the above-mentioned subjects, there is provided a method for rapidly purifying a polluted object comprising the steps of: a) making and maintaining high-concentration ozonized water which has at least 20 ppm ozone in solution in water by continuously contacting and mixing by stirring a predetermined constant amount of water with an excess amount of gas, wherein said gas includes gaseous ozone of which concentration is predetermined and kept at a constant value, and wherein said contacting and mixing by stirring is continued for at least a time period which is required by said solution to reach equilibrium concentration, under the conditions of constant temperature and pressure, in a substantially completely closed chamber; and b) supplying said high-concentration ozonized water out of said chamber to said polluted object.

Preferably, said temperature is under about 5 ° C and said constant amount of water is pure water.

Further preferably, said step b) further includes a step of diluting said high-concentration ozonized water.

The method of the present invention is further preferred if said polluted object is polluted by amoebas with cysts which need to be rapidly disinfected.

The method of the present invention is further preferred also if said polluted object needs disinfection of animal plankton larvae.

The method of the present invention is further preferred also if said polluted object is a surface of a industrial product which accompanies unnecessary adherent organic substances.

In general, ozone generating methods are broadly classified into an electric discharge method and an electrolysis method. The electric discharge method is less expensive, but requires a high voltage, and nitrogen oxides are often present. Therefore, its practical application to the clinical field is comparatively undesirable from the standpoint of safety. In contrast therewith, the electrolysis method does not require a high voltage, and the presence of nitrogen oxides is not observed. Thus, the electrolysis method is suitable for a clinical use.

From the electrolytic cell for the generation of ozone there stably issues an ozone-containing oxygen gas of 10% or so in a steady state. But just after application of power the amount of ozone generated is small. Thereafter, the amount of ozone generated increases gradually, and a steady state is reached in half an hour or so, followed by stable generation of gas, which is continued. Thus, the supply of electricity is required continually for concentration control, and the stability of the cell is enhanced thereby.

Ozone is an oxidizing agent extremely high in activity inherently and is essentially very unstable. Consequently, ozonized water is also unstable, and at ordinary temperatures the concentration of ozone drops spontaneously in a half-life period of a little less than one hour. For this reason the storage of ozonized water has been considered impossible. Ozone is difficult to dissolve in water, as reflected in a maximum concentration of 10 ppm or so at an ordinary temperature. The foregoing spontaneous concentration dropping phenomenon is also concerned in the reason why the concentration of ozone varies depending on dissolving conditions.

Therefore, with the conventional way of thinking, a strict concentration control is next to impossible, and it is a matter of course that it has so far been impossible to obtain a practically employable product.

Application of the method of controlling the concentration of a gas-in-liquid solution indirectly according to the present invention to the foregoing ozonizer has shown that excellent stability and reproducibility are ensured and that all of the conventional problems can be eliminated and an ideal performance attained. By the present invention, moreover, various basic properties of ozone so far unknown has been made clear, and a lot of important and practically essential results has been obtained.

Thus, according to the present invention, very fine organisms such as protozoa and plankton, as well as bacteria, virus, or others which they are symbiotic with or parasitic on, or oils, fats, or other pollutants, deposited on materials, are clarified rapidly and safely. Preferably, in subjecting very fine organisms, bacteria, virus, or others with which or on which they are symbiotic or parasitic, as well as oils, fats, resins, or other pollutants, to the action of the ozonized water, there is preferably adopted a light physical stimulation such as ultrasonic vibration, application of a water jet, a mechanical friction, or the like.

As to the temperature dependence of the ozonized water concentration, there have been many uncertain points heretofore, but clear results are obtained by a stable reproducibility. A summary of this point is as shown in experiment A1 which will be described later. By diluting a high-concentration solution thus prepared with water to an appropriate level there is obtained ozonized water having a required concentration accurately in a simple manner and in high reproducibility. Data based on this method and applicable to practical apparatus are also shown in experiment A1. Ozonization of an isotonic sodium chloride solution, which has heretofore been considered impossible, can be effected by this method according to the present invention. This is as shown in experiment A2, and the results of ozonization of buffer solutions are as shown in experiment A3, which will be described later.

All of the above results, which have heretofore been considered impossible or uncertain, correspond to new findings, which were attained and made clear for the first time by the excellent accuracy, stability and reproducibility according to the method of the present invention. Experiments A, which will be described later, demonstrate the excellent performance of the method of the present invention as a great advantage in practical use.

In order to make or maintain high-concentration ozonized water, such technique illustratively may comprise an indirect control method of Japanese Patent Application No. H5-282035 (Laid Open No. H7-112123) and preferably the following improvements.
1) As the water for dissolving ozone there is used pure water with substances little dissolved therein.
2) The temperature of ozonized water is to be rendered as low as possible.

With this technique, the decomposition and consumption of ozone in a high-concentration ozonized water can be kept to a minimum.

Next, methods and effects of supplying the high concentration ozonized water to the polluted objects to be purified according to the present invention suitable for respective types of the polluted objects follow as discussed below.

### 1. Rapid Sterilizing Effect against Amoeba.

In an in vitro experiment, amoeba trophozoite, or vegetative hypha, was destroyed in an instant with 4 ppm ozonized water, which ozonized water, however, was ineffective against the cyst of the same amoeba. The details of this point will be described later.

The cyst has a strong outer membrane (encystment) and thus the resistance thereof is enhanced, so the following combination is adopted as the method for introducing ozone through such strong membrane into the interior and causing the cyst to die out.
1) Supplying the high-concentration ozonized water to increase the space density in the membrane destroying reaction.
2) Applying a weak physical stimulation such as ultrasonic vibration, water jet, or mechanical friction, to accelerate the separation of destroyed membrane.

When the above method was applied to amoeba cyst, it turned out that the cyst dies up with the high-concentration ozonized water alone. The details of this result will be mentioned later.

### 2. Removal of Photoresist on Silicon Wafer.

Since the amoeba cyst is enclosed with a hard membrane, it can be considered to be a model of pollutant deposited on the surface of an implement. In view of this point, the foregoing surface purifying method has been tried using as an object of experiment a photoresist-coated silicon wafer as an example of a structure the whole surface of which is coated with a resin film.

As a result of the experiment it turned out that the photoresist is removed easily after treatment with a high-concentration ozonized water as referred to above in 1) and by a light physical stimulation such as ultraviolet vibration, mechanical friction, or water jet, as referred to above in 2). The details of this point will be mentioned later.

The application of this method is not limited to the one just shown above, but this method can be a general method applicable widely to the removal of pollutants adhered to the surfaces of implements.

### 3. Brine Shrimp Insecticide.

The surface structure of the above photoresist-coated wafer is rather similar morphologically to the surfaces of Crustacea organisms which coat hard shells. In this connection, using larvae of brine shrimp (Artemia) as a crustacean, an experiment has been conducted to check the influence of treatment with a high-concentration ozonized water upon the said larvae.

The results of the experiment show clearly that the larvae can be killed easily merely by using a high-concentration ozonized water as referred to above in 1) and that any other operation is not necessary. The details of this point will be described later.

The Artemia used in this experiment is known to be an animal species strong in both vital power and resistance. The fact that the Artemia could be killed easily with the high-concentration ozonized water shows that the use of the high-concentration ozonized water is effective in killing animal plankton larvae at large and also effective for the purpose of purification and management of environment. Related concrete works are also very easy and inexpensive.

In the following experiments A, using repeatedly a single electrolytic type ozonizer capable of generating 0.4 g of ozone per hour, it was confirmed that a steady state free of change in the ozone generating capacity at every repetition was consistent. In this state the concentration of ozone contained in the generated oxygen was 10%. For the dissolution of ozone into water there is adopted a simple bubbling method in a free air flow state in the case where the vessel used is an open vessel, while in the case of a semi-closed vessel a convection space for the ozone-containing oxygen is formed above the water surface in the vessel. But in the method used herein there is used a completely closed vessel in which an upper space is a gas-phase space of the ozone-containing oxygen, and dissolving and stirring are performed continually. For determining the amount of ozone dissolved in water there was adopted the titration method using KI-starch described in the Japanese Pharmacopoeia.

### [Experiment A1] Temperature Dependence of Ozone Concentration.

In the preparation of ozonized water there appears a marked difference in the results obtained between different manufacturing processes even if the processes employ the same ozone generating cell under the same conditions. For example, when saturation concentrations were determined at a water temperature fixed to 25 °C, the results obtained were 1.50 ppm in the case where the vessel used was a complete open vessel, 5.00 ppm in a semi-open vessel and 14.25 ppm in a completely closed vessel used in the method of the present invention.

Next, in the method of the present invention, the temperature set for the solution was reduced stepwise and the saturation concentration at each temperature was determined, the results of which are as follows:

| Temperature (°C) | Ozone Concentration (ppm) |
|---|---|
| 0 | 38.75 |
| 5 | 36.00 |
| 10 | 25.00 |
| 15 | 18.00 |
| 20 | 15.00 |
| 25 | 14.25 |

In the above table, the temperature control accuracy is within ±0.1°C at each temperature, and the ozone concentration is a mean value of values obtained in three measurements. The above data indicate a remarkable increase of ozone concentration at low temperatures.

Further, with respect to each of the temperatures adopted in the above experiment A1, the same temperature was kept constant for five hours while the solution was allowed to stand under continued dissolving and stirring, and the change in ozone concentration was observed at every one hour. As a result, variations in the ozone concentration were within ±3% in the case where the solution temperature was not lower than 20°C and within ±1% in the case where the solution temperature was not higher than 5°C, thus suggesting that the temperature control at room temperature and thereabouts is relatively difficult.

The high-concentration ozonized water obtained at each low temperature mentioned above can be adjusted low in concentration by dilution. In the case where the dilution is made in an ordinary vessel such as a beaker, there may be used a calculated value, but in the case where the dilution is performed using a long flow path, the dilution ratio may be set taking into account a drop of concentration in the flow path. In the dilution to the usual concentration of 4.00 ppm in hand disinfection, the error was within ±0.2 ppm and thus the reproducibility was good.

The results of the above experiment A1 clearly show that in the region not attainable by the conventional method the method of the present invention exhibits such outstanding effects as:
1) high solubility,
2) remarkable increase in the dissolved ozone concentration at low temperatures, and
3) stable continuation of saturation concentration, and that the method of the invention is very advantageous in practical application.

### [Experiment A2] Ozonization of Isotonic Sodium Chloride Solution.

Heretofore it has been said that the ozonization of an isotonic sodium chloride solution is impossible. But we were the first to find out that the said ozonization can be effected by using the method of the present invention. As to saturation concentration, it was determined during operation in accordance with the method of the present invention in the same manner as above, while the value of a half-life period was obtained by checking the drop of concentration with the lapse of time after discontinuance of the operation and by subsequent calculation.

As is the case with pure water, the saturation concentration of ozone depends on the water temperature, but the degree of the dependence is relatively small. More specifically, at room temperature the saturation concentration is 10.38 ppm and in an ice-cold condition it is 16.29 ppm. But the half-life period is extremely short. At room temperature it is 11.2 minutes and attenuates rapidly, with subsequent maintenance at around 1.0 to 1.2 ppm for several hours. Also under an ice-cold condition the half-life period is short and attenuates rapidly, but with subsequent maintenance at around 3.0 ppm for over 7 hours.

Such extremely short half-life periods are characteristic of an isotonic sodium chloride solution, and for this reason the ozonization thereof has heretofore been considered impossible. But this is a misunderstanding. According to the method of the present invention, since the dissolving efficiency is much higher than in the conventional method, it was possible to confirm the foregoing fact, namely the fact that the ozonization of the isotonic sodium chloride solution can be done. The saturation concentration is stable during application of the method of the present invention.

### [Experiment A3] Ozonization of Buffer Solution.

There are a wide variety of buffer solutions, but here will be shown a part of experimental examples on the ozonization of phosphoric acid buffer solutions used frequently in the realm of biology. Also as to phosphoric acid buffer solutions, they are classified into various types, of which the following two types will be outlined below.
1)

| | |
|---|---|
| Na₂ HPO₄ (anhydrous) | 5.7 g |
| KH₂ PO₄ (anhydrous) | 3.6 g |
| | ; pH 7.0 |

2)

| | |
|---|---|
| NaCl | 8.0 g |
| KCl | 0.2 g |
| Na₂ HPO₄ (anhydrous) | 1.15 g |
| KH₂ PO₄ (anhydrous) | 0.2 g |
| | ; pH 7.4 |

Saturation concentration and half-life period were determined in the same way as in the previous experiments.

### Buffer solution 1):

At room temperature, saturation concentration was 5.00 ppm, and half-life period was 1.22 hr.
At ice-cooled temperature, saturation concentration was 28.25 ppm, and half-life period was 1.20 hr.

### Buffer solution 2):

At room temperature, saturation concentration was 8.00 ppm, and half-life period was 12.7 min.
At ice-cooled temperature, saturation concentration was 22.25 ppm, and half-life period was 2.37 hr.

The buffer solution 2) is extremely short in its half-life period at room temperature and is similar in this point to the isotonic sodium chloride solution because of incorporation therein of both NaCl and KCl. In an ice-cold condition, however, the half-life period thereof is longer than that of the buffer solution 1).

Irrespective of temperature both buffer solutions exhibited little change in pH with dissolution of ozone, and there was no change in their buffering capacity.

The above knowledge was obtained for the first time by application of the method of the present invention to the field so far free of any measurement example because of ozonization having been considered impossible. During application of the method of the present invention there is recognized no change in saturation concentration even with the lapse of time, thus permitting use of a buffer solution which is constant in its ozone concentration.

If there is used an ozone generating cell different in capacity from that used in the above experiments A, there will be obtained different values of saturation concentration from those mentioned in the experiments A. For example, if there is used an electrolytic type cell capable of generating 0.8 g of ozone per hour, the saturation concentration values shown above will increase several ten percent in comparison with the original values. This is because of increase of ozone concentration in the electrolytic oxygen.

In a later [Experiment B1], we will show that when the two conditions of using pure water 1) and using a low water temperature 2) were well satisfied, there was confirmed a highest ozone concentration of not lower than 80 ppm and thus a much higher concentration than the conventional maximum concentration was recorded in the history of ozonized water. This experimental fact demonstrated the propriety of our design idea.

As a result, it has become possible to supply an high concentration ozonized water having a much stronger reactivity than before and greatly expand the application range of ozonized water as will be shown below:

### 1. Sterilizing Effect against Amoeba.

### 1) Amoeba Trophozoite and Low-Concentration Ozonized Water.

It has become clear through an in vitro experiment that amoeba trophozoite, like bacteria, dies out almost in an instant by only a simple treatment using 4 ppm ozonized water. Morphologically, amoeba is evidently larger in size than bacteria, and for this reason it had been presumed that the insecticide thereof would require a high-concentration of ozonized water. But the fact was contrary to the expectations. As is the case with bacteria, 4 ppm was sufficiently effective. However, with increase in worm size, one amoeba individual consumes a larger amount of ozone than a volume proportion value in an instant.

Further, such a rapid amoebacidal action mechanism using 4 ppm ozonized water has been made sure as follows through a sterilizing experiment conducted under a microscope. More specifically, the destruction of cell membrane induced instantaneously by contact with ozonized water gives rise to cytolysis. This shocking cell destruction is a fact also coincident with the destruction phenomenon of Bacillus subtilis by 4 ppm ozonized water which has been recognized in an electron microscope observation. Thus, it is a phenomenon which characterizes the action of ozonized water on organisms, and for the first time this point was confirmed and recorded. The details of the related experiment are as shown in [Experiment B2].

### 2) Amoeba Cyst and Low-Concentration Ozonized Water.

As mentioned above, the amoeba trophozoite could be killed and extinguished easily with 4 ppm ozonized water. But it turned out that the amoeba cyst displayed a strong resistance to 4 ppm ozonized water and that the sterilizing effect was almost zero percent, that is, almost all of the amoeba cysts subjected to the experiment survived. The details of this point are as described in [Experiment B2].

The results of this experiment show that the strong extine form by amoeba for the purpose of resisting the worsening of the growth environment plays a role of protection satisfactorily also against ozone which is rare in the ordinary natural environment. This fact is in agreement with the fact that there have been few successful examples in the development and research of amoeba exterminating drugs so far conducted, which fact is attributable to the aforesaid strong extine.

Thus, for achieving the purpose of killing the amoeba cyst through such extremely strong protective wall, there has arisen the necessity of practically using a high-concentration ozonized water as a drug having a more powerful action.

### 3) Amoeba Cyst and High-Concentration Ozonized Water.

For the reason stated above we have tried using a high-concentration ozonized water having the most powerful sterilizing action. As a result of an in vitro experiment it turned out that 40 ppm ozonized water could decrease the percent survival of the cyst to the 10% mark by only a 30 second contact. This means that the sterilization of the cyst which has heretofore been considered almost impossible is completed in a simple manner by only one minute treatment with the high-concentration ozonized water at an ordinary temperature and that a rapid and thorough purification can be attained. The details of this experiment are as described in [Experiment B2].

Regarding this sterilizing mechanism, as a result of having observed changes in properties and form of the cyst, using 40 ppm ozonized water, under an optical microscope, there were recorded changes with time in the state of reaction into ozone, including differences between young cysts and matured cysts. In conclusion, young cysts, like trophozoite, burst in an instant, while as the degree of maturation becomes higher, the resistivity increases. However, it turned out that in the 40 ppm ozonized water even matured cysts undergo atrophy and their vital activity stops within one minute.

From the above facts it is seen that if a high-concentration ozonized water of 40 ppm or higher is used in an exposure time of several minutes, it is possible to effect a nearly perfect extermination. Thus, the sterilization of the cyst which has so far been extremely difficult became possible in a simple and perfect manner by the use of a high-concentration ozonized water.

The effectiveness of a high-concentration ozonized water against amoeba confirmed by this experiment has also been made sure by the fact that when a high-concentration ozonized water was applied to a cooling tower for air conditioning, it was possible to sterilize and disinfect both amoeba as host and Legionella strain as pathogenic bacterium at a time and thereby prevent Legionella infection completely. A further effect involves rapid purification for the related water-passage environments to prevent recurrence of this type of microorganism contamination.

The above experimental fact shows that the occurrence of Legionnaire s disease can be prevented easily by using a high-concentration ozonized water. In this case, the water passage for air conditioning, including the water flowing through the interior, can be purified with ozone and there is no fear of chemical contamination recognized in the use of other drugs.

On the other hand, amebic dysentery also forms cyst, and matured cyst discharged from a cyst carrier shifts orally into the body of another person through an implement, hand or fingers of the person, etc., whereby the person is infected and attached with the disease. Thus, since amoeba can take the form of cyst which is highly resistant, it has heretofore been considered extremely difficult to develop an effective drug.

Therefore, the effectiveness of rapid, perfect and easy disinfection using ozonized water which can annihilate even cyst within one minute and which was confirmed by this experiment, is expected to greatly contribute to the prevention of this type of diseases.

### 2. Effect of Removing Photoresist on Silicon Wafer.

If the high-concentration ozonized water is supplied to a silicon wafer having one side coated with photoresist, the surface of the photoresist film undergoes a slight change in several seconds, the surface smoothness is lost and there appear fine wrinkles and pores. This change grows more intense with the lapse of time. This phenomenon can be traced with the naked eye on the basis of changes in reflected light. However, with only this contact of the high-concentration ozonized water with the silicon wafer, there will not occur exfoliation of the photoresist film.

If this ozonized water treatment is followed by the application of a weak ultrasonic vibration, the destructed film of the photoresist will come off the wafer easily while being broken into fine pieces, leaving a clean silicon surface. Alternatively, if the photoresist film is subjected to ozonolysis by an extremely light mechanical friction or manual rubbing or by a jet of water, it will easily come off the wafer surface while being divided into fine pieces as in the ultrasonic radiation, whereby the underlying silicon surface is exposed as a clean surface. A fact that the silicon surface treated by this treatment is in the same structural state as a clean silicon surface not coated with the resin film, has been confirmed spectroscopically by FTIR measurement. The details of this experiment will be described later in [Experiment B3].

The results of this experiment show that an oxidative purifying action of the high-concentration ozonized water is extremely strong. It has become clear that the resin film easily undergoes an oxidative destruction by merely being dipped in the ozonized water at a low temperature in a short time and that it can be removed easily by a light physical stimulation. With these features, the ozonized water proved to be employable effectively in the semiconductor manufacturing process and the like.

Such film destructing and eliminating action of the high-concentration ozonized water is based on the strong oxidative destruction action of the ozonized water which action can be exerted not only on the resin film but also on organic films at large, including oil and fat films. It is a characteristic peculiar to ozone. According to this principle, concrete materials capable of being subjected to the rapid purification using such a high-concentration ozonized water are not limited to silicon surfaces but cover various other materials, including glass and ceramic materials. Organic contaminant films on these materials can also be removed rapidly to a thorough extent.

That is, the rapid purifying method using a high-concentration ozonized water is applicable to all of the fields in which a wet washing method using strong acid, strong alkali and hydrogen peroxide has heretofore been adopted frequently. The purifying method in question does not rely on acid or alkali but is conducted in a neutral condition, so the application thereof can be extended also to other objects than those mentioned above. For any object to be purified the purifying method in question can exhibit such features as easy and simple execution of works, thorough purification, low temperature, short time, easy post-treatment, and low cost. Further, this purifying method is harmless to the environment.

### 3. Brine Shrimp Insecticide.

Brine shrimp is a lower Crustacea grown in salt water and generally indicates one belonging to the Artemia genus. Dried spawns thereof, when immersed in sea water, hatch out into nauplius larvae in a few days, which are used not only as feed to marine fish and shellfish fries but also as test materials for radiant rays and poisonous substances.

In this experiment there were used nauplius larvae obtained by hatching dried Artemia spawns from the U.S. in 2% brine at 29° C. The high-concentration ozonized water used was very effective. In an insecticidal experiment conducted on the first day of the hatching all the nauplius larvae died within one minute at an ozone concentration of not lower than 30 ppm, while at an ozone concentration of 20 mm the activity of the nauplius larvae subsided and all died after three hours. Also in insecticidal experiments conducted on the second and subsequent days after the hatching there were obtained similar results. During the period of several days after the hatching during which the nauplius larvae carried yolks there was recognized no difference in resistance as they grew. The details of this experiments will be described later in [Experiment B4].

In pure water, as will be mentioned later in [Experiment B1], the concentration of the high-concentration ozonized water reaches 80 ppm or more, and when the ozonized water is diluted twice there is obtained a concentration of 40 ppm. But these concentrations are dependent on both water quality and water temperature. Concentration attenuation is rapid particularly in a brine. For this reason it has heretofore been said that ozone does not dissolve in an isotonic sodium chloride solution (brine). But this is a misunderstanding based on the actual fact that the concentration attenuation is rapid at an ordinary temperature and that with decrease in partial pressure in a gas phase the attenuation is markedly accelerated to an unmeasurable extent. Since the brine used in this experiment is 2% brine, the concentration of ozone in the high-concentration ozonized water used drops to a greater extent than the preset dilution ratio. But there is taken a form such that from this initial time point the nauplius larvae come into contact with the high-concentration ozone and that the ozone concentration then drops with the lapse of time. The foregoing ozone concentration of 30 or 20 ppm indicates such an initial concentration of the diluted ozonized water. Although the ozone concentration continues to drop with the lapse of time, an initial concentration of 30 ppm in brine can be ensured by adopting the method using a high-concentration ozonized water of 80 ppm in pure water.

It is not until adoption of the above dilution method that it becomes possible to conduct a quantitative experiment. In this sense it is required that the concentration of the high-concentration ozonized water for dilution be as high as possible.

The results of this experiment suggest an actual possibility of causing animal planktons to die out even in sea water. It follows that a concrete method for environment purification or appropriate environment management can be established. For example, if the method of the present invention is applied to a plant cooling water to completely kill organisms or larvae adhered to the water passage walls in the plant, it becomes possible to prevent an increase in the resistance of flowing water caused by adhesion of such organisms or larvae to the walss and thereby maintain the normal plant cooling function. Since the organism annihilation is completed in a short time, if a calculated amount of an oxidizable substance is added just thereafter, surplus ozone will become extinct in an instant. Thus, there is no chemical influence on a heat exchanger, etc., nor is there any influence on the environment.

Further, since bacteria can be sterilized easily at a low ozone concentration of 4 ppm, a fractional purification for killing bacteria alone and leaving plankton alive in a coexistent system of bacteria and plankton, can be done by merely selecting a suitable ozone concentration in ozonized water.

Such a technique has heretofore been impossible, so the study in question is very significant in point of conservation, purification and management of the environment.

### [Experiment B1] Preparation of High-Concentration Ozonized Water.

In connection with the aforementioned method for preparation of high concentration ozonized water, the following improvements are further added.
1) As the water to be used for dissolving ozone there is used a pure water of a high purity obtained by passing water through filter membrane, active carbon and ion exchange resin.
2) The temperature of ozonized water is reduced to a level not higher than 5°C.

As an ozone generating cell there is used an electrolytic type cell, and 10% pure ozone in oxygen not containing impurities such as nitrogen oxides is used. As an ozone dissolving tank there is used a tank of a circulative dissolving type having a circulation path which goes through an ozone dissolver. The whole of this dissolving system is set at a temperature not higher than 5°C. In one hour after the supply of electricity the concentration of ozone in water reached 83 ppm and thereafter was maintained at this concentration level automatically.

The ozone concentration was determined by redox titration in a microburette using a sodium thiosulfate normal solution with KI-starch as an indicator. This titration method is a basic method for the determination of an oxidative substance, in which the judgment of an end point is sharp.

A high concentration of the 80 ppm mark which has heretofore been unattainable has become maintainable constantly.

### [Experiment B2] Sterilizing Effect of High-Concentration Ozonized Water against Amoeba.

1) 200 µ l of 4 ppm ozonized water is added to 40 µ l of an Acanthamoeba trophozoite suspension (worm concentration 6.2 × 10⁴ cells/ml), and 15 µ l of a terminator (bovine serum) is added at every 1, 3, 5, 10 and 30 seconds to terminate the reaction. A predetermined amount of the reaction solution is sampled as a test solution after stirring to render it homogeneous and is determined for percent survival (%) using an optical microscope in accordance with a dyeing method. Mean values in three continuous runs of this experiment are shown in Table 1. Thus, with the 4 ppm ozonized water, the amoeba trophozoite dies almost completely within one second. In all the other measurement times than 0 second (experiment start point) the percent survival is almost zero and the deviation is also small.

**Table 1**

| Change with time in percent survival of trophozoite (4 ppm ozone) | | | | | | |
|---|---|---|---|---|---|---|
| Time (sec) | 0 | 1 | 3 | 5 | 10 | 30 |
| PercentSurvival (%) | 96.27 | 0.16 | 0.00 | 0.01 | 0.00 | 0.44 |
| Standard Deviation | 1.42 | 0.27 | 0.00 | 0.02 | 0.00 | 0.20 |

It was confirmed in an insecticidal experiment under a microscope that the mechanism of such a rapid amoebacidal action using 4 ppm ozonized water corresponds to cytolysis based on the destruction of cell membrane.
2) Using an Acanthamoeba-cysts suspension (6.0 × 10⁴ cells/ml), changes in the percent survival with time are determined in the same way as is the case with trophozoite, provided the reaction times are set at 5, 10 and 30 seconds and 10 minutes. Mean values obtained in three continuous runs of this experiment are shown in Table 2. The resistance of the cyst is strong, and under these conditions there is little insecticidal effect.

**Table 2**

| Change with time in percent survival of cyst (4 ppm ozone) | | | | | |
|---|---|---|---|---|---|
| Time (sec) | 0 | 5 | 10 | 30 | 600 |
| PercentSurvival (%) | 99.87 | 99.47 | 99.87 | 99.80 | 99.10 |
| Standard Deviation | 0.12 | 0.47 | 0.23 | 0.20 | 0.01 |

3) Using an Acanthamoeba-cyst suspension (6.0 × 10⁴ cells/ml) and ozonized water of 40 ppm, changes in the percent survival with time are determined in the same way as is the case with 4 ppm ozonized water, provided the reaction times were set at 3, 5, 10 and 30 seconds. Mean values obtained in three continuous runs of this experiment are shown in Table 3. With 40 ppm ozonized water, unlike 4 ppm ozonized water, about 85% cysts died in 30 seconds, thus proving effectiveness of the high-concentration ozonized water.

**Table 3**

| Change with time in percent survival of cyst (40 ppm ozone) | | | | | |
|---|---|---|---|---|---|
| Time (sec) | 0 | 3 | 5 | 10 | 30 |
| Percent Survival (%) | 99.87 | 96.70 | 72.67 | 32.30 | 16.00 |
| Standard Deviation | 0.12 | 0.92 | 3.16 | 9.15 | 3.96 |

Thus, by using a high-concentration ozonized water of 40 ppm or more in an exposure time of several minutes, it is made possible to cause the cysts to die almost completely. The sterilization of cyst, which has heretofore been extremely difficult, can now be done to a perfect extent by using such a high concentration ozonized water.

### [Experiment B3] Effect of Removing Photoresist on Silicon Wafer.

A silicon wafer coated on one side with photoresist is cut into 1 cm square pieces as test pieces. Then, three such test pieces are placed with the photoresist side up in a prepurified petri dish, into which is then poured slowly 10 ml of 40 ppm ozonized water prepared under cooling. The petri dish is then covered and allowed to stand. During this ozone treatment the temperature of the ozonized water held at 10°C, and in several seconds and thereafter the pouring of the ozonized water slight changes on the photoresist film surface are recognized by reflected light, which changes are intensified with the lapse of time. Upon lapse of each of 1, 2, 4, 8 and 15 minutes after the pouring of the ozonized water is decanted off and washed with two 5 ml portions of pure water, then air-dried.

With only the contact with the high-concentration ozonized water it can merely be seen that the photoresist film is wrinkled and cracked irregularly and that an oxidative destruction with ozone has extended to the resin film. But the exfoliation of the resin film cannot be confirmed with the naked eye. The FTIR spectrum thereof exhibits a clear change in comparison with the standard spectrum of untreated photoresist surface. But the resolution is poor and it is impossible to make analysis, although the appearance of an oxidative change was demonstrated clearly. At this time, however, there scarcely occurs any spontaneous exfoliation of the photoresist film.

Subsequently, the following three kinds of post-treatments are applied each independently to the ozone-treated test pieces.

### 1) Radiation of Ultrasonic Wave.

When a physical vibration is imposed on each test piece by radiation of a weak ultrasonic wave, the destructed photoresist film is easily exfoliated while being broken into fine pieces, resulting in exposure of a clean silicon surface. This exposed surface has a colorless luster, and the FTIR spectrum thereof is coincident with the standard silicon spectrum, thus proving complete exfoliation of the photoresist coating. But as to the test piece subjected to one minute ozone treatment, the exfoliation of its destructed resin surface was partially incomplete.

### 2) Weak Mechanical Friction.

When the oxidative-destructed resin surface is rubbed lightly by hand using a spatula, the destructed resin film is wholly exfoliated while being broken into small pieces, resulting in exposure of a clean silicon surface. Like the preceding case, all the other test pieces than the one subjected to one minute ozone treatment exhibit a perfect exfoliation of their resin films in terms of FTIR.

The same results as above are obtained also when rubbing is made lightly with any other solid edge than a spatula.

### 3) Water Jet.

When water is jetted to the ozone-treated resin film surface of each test piece from a fine hole formed by drawing a glass tube, the photoresist film which has undergone ozonolysis is exfoliated easily while being divided into fine pieces, resulting in exposure of the underlying silicon surface as a clean surface. Like the preceding case, the FTIR spectrum thereof demonstrated a perfect exfoliation of the destructed resin film.

In conclusion, when each test piece is immersed two minutes in 40 ppm ozonized water, the photoresist film thereof undergoes oxidative destruction, and the destructed film is exfoliated easily by the application thereto of a weak physical stimulation.

### [Experiment B4] Insecticidal Effect against Brine Shrimp (Artemia).

40 mg of dried Artemia spawns from the U.S. are weighed into a 200 ml Erlenmeyer flask, into which is then added 100 ml of 2% NaCl solution preadjusted to 29° C. The flask is then placed in a 29° C water bath while the passing of air is continued.

In 30 hours, a middle layer of a suspension of nauplius larvae after hatching and leaving the testa is sampled with pipette and transferred into a test tube, into which is then added 40 ppm ozonized water in a quantitative manner. Initial dilution concentrations are set to 36 ppm, 30 ppm, 20 ppm, 10 ppm and 4 ppm for such test tubes, which are then allowed to stand at room temperature. As a control, distilled water is added in the same amount, and the influence of a change in th-e concentration of salts was observed. In the two samples of 36 ppm and 30 ppm, all the nauplius larvae die in an instant simultaneously with the pouring of the respective ozonized waters and sink to the bottom of the test tube, in a period of time not longer than one minute or so. In the sample of 20 ppm, although some survive, all the larvae die in 3 hours and are deposited on the test tube bottom. At the ozone concentrations not higher than 10 ppm there was recognized survival of all the larvae.

In 43 hours after the hatching, the nauplius larvae grow more than the previous day and their motions become active. Like the previous 30 hour case there was conducted ozone treatment to afford about the same results, but the time required for the development of ozone effect was about five times later than in the previous case. In 67 hours after the hatching there was observed a further growth, although the results of ozone treatment were almost the same as in the 43 hour case after hatching.

To conclude, at an ozone concentration (initial dilution concentration) of not lower than 30 ppm all the nauplius larvae of Artemia die within one minute, while ozone concentrations (initial dilusion concentrations) not higher than 10 ppm are not effective. Thus, for killing brine shrimps in a brine it is necessary that the initial ozone concentration be high. To this end it is absolutely necessary to prepare a high-concentration ozonized water.

According to the rapid purifying action of the high-concentration ozonized water prepared by the method of the present invention, a wide variety of very fine organisms, including bacteria, virus, amoeba and crustacean larvae, can be sterilized, inactivated and killed, and films and stains of resins, oils and fats can be destructed and exfoliated rapidly. Besides, the method of the invention such features as easy execution of works, thorough purification, low temperature, short time, easy post-treatment and low cost. Further, it is very safe for the environment, thus having excellent utility.

The method of the invention covers an extremely wide range of application fields, including the field of public hygiene such as the prevention of infectious diseases, the field of precision industry requiring a thorough purification such as the semiconductor manufacturing process, plant industry requiring a large amount of water, and fields related to the improvement of environmental pollution and environment management and related to the conservation of water environment such as the water quality control in farming fishery and various fresh food markets. Great actual profits are promised in these fields.

## Claims

1. A method for rapidly purifying a polluted object comprising the steps of:
a) making and maintaining high-concentration ozonized water which has at least 20 ppm ozone in solution in water by continuously contacting and mixing by stirring a predetermined constant amount of water with an excess amount of gas, wherein said gas includes gaseous ozone of which concentration is predetermined and kept at a constant value, and wherein said contacting and mixing by stirring is continued for at least a time period which is required by said solution to reach equilibrium concentration, under the conditions of constant temperature and pressure, in a substantially completely closed chamber; and
b)supplying said high-concentration ozonized water out of said chamber to said polluted object.

2. The method as set forth in claim 1, wherein said temperature is under about 5°C and said constant amount of water is pure water.

3. The method as set forth in one of claims 1-2, wherein said step b) further includes a step of diluting said high-concentration ozonized water.

4. The method as set forth in one of claims 1-2, wherein said polluted object is polluted by amoebas with cysts which need to be rapidly disinfected.

5. The method as set forth in one of claims 1-2, wherein said polluted object needs disinfection of animal plankton larvae.

6. . The method as set forth in one of claims 1-3, wherein said polluted object is a surface of a industrial product which accompanies unnecessary adherent organic substances.

7. The method as set forth in claim 6, wherein said surface is a surface of a silicon wafer.
